# EUROPEAN PATENT APPLICATION

(11) **EP 4 473 927 A1**
(43) Date of publication of application: **11.12.2024**
(21) Application number: 24179743.0
(22) Date of filing: 04.06.2024
(51) Int. Cl.: A61B 18/14

(54) **MEDICAL PROBE END EFFECTOR WITH HINGES**

(30) Priority: 05.06.2023 US 202363506275 P; 06.05.2024 US 202418656377
(71) Applicant: Biosense Webster (Israel) Ltd., 2066717 Yokneam (IL)
(72) Inventor: AGNEW V, William James, Irvine, 92618 (US); THALER, Sean D., Irvine, 92618 (US); VAN NIEKERK, Pieter Emmelius, Irvine, 92618 (US); RODRIGUEZ, Jasson, Irvine, 92618 (US)
(74) Representative: Carpmaels & Ransford LLP

(57) **Abstract**

The disclosed technology includes a medical probe including a tubular shaft and an end effector. The tubular shaft can define a longitudinal axis and have a tubular shaft end. The end effector can be coupled to the tubular shaft end. The end effector can include a crown. The end effector can include at least one spine that extends along the longitudinal axis. The at least one spine can include a deployed position and delivery position relative to the longitudinal axis. The at least one spine can include a first material. The end effector can include at least one hinge coupling the at least one spine to the crown. The at least one hinge can include a second material different from the first material.

## Description

### CROSS REFERENCE TO RELATED APPLICATIONS

The application claims benefit of priority to prior filed U.S. Provisional Patent Application No. 63/506,275 filed June 5, 2023 (Attorney Docket No.: BIO6844USPSP1 - 253757.000370), which is hereby incorporated by reference in full herein.

### FIELD

The present invention relates generally to medical devices, and in particular basket catheters configured to transition between a collapsed configuration and an expanded configuration.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching ends of the linear spines to a tubular shaft (e.g., a pusher tube) to form a spherical basket. Some basket catheters can take up a lot of space and be difficult to insert through an insertion catheter. Furthermore, due to the configuration of the basket and/or metal fatigue, it is possible for the spines to break when being inserted or retracted through the sheath or manipulated over a period of time. Accordingly, there is a need in the art for alternative designs of basket catheters that help to reduce the likelihood that a spine will break. This and other issues can be addressed by the technology disclosed herein.

### SUMMARY

The disclosed technology includes a joint of an end effector of a medical probe. The end effector can comprise a crown, at least one spine, and at least one hinge. The at least one spine can extend along a longitudinal axis, comprise a deployed position and delivery position relative to the longitudinal axis, and comprise a first material. The at least one hinge can couple the at least one spine to the crown and comprise a second material different from the first material.

The disclosed technology includes a medical probe. The medical probe can comprise a tubular shaft and an end effector. The tubular shaft can define a longitudinal axis and have a tubular shaft end. The end effector can be coupled to the tubular shaft end. The end effector can comprise a crown, at least one spine, and at least one hinge. The at least one spine can extend along the longitudinal axis, comprise a deployed position and delivery position relative to the longitudinal axis, and comprise a first material. The at least one hinge can couple the at least one spine to the crown and comprise a second material different from the first material.

The disclosed technology includes a medical probe. The medical probe can comprise a tubular shaft and an end effector. The tubular shaft can define a longitudinal axis and have a tubular shaft end. The end effector can comprise at least one spine extending along the longitudinal axis, comprise a deployed position and delivery position relative to the longitudinal axis, and comprise a first material. The at least one hinge can couple the at least one spine to the tubular shaft end and comprise a second material different from the first material.

The disclosed technology can include a method of constructing a joint of an end effector. The method can comprise positioning a tube over a portion of a spine or a portion of a crown. The method can comprise positioning the crown and the spine proximal one another so as to define a gap. The method can comprise positioning the tube over the gap. The method can comprise heating the tube. The method can comprise, in response to the heating, shrinking the tube to cover a spine distal end of the spine and a crown end portion of the crown.

The disclosed technology can include a method of constructing a joint of an end effector. The method can comprise positioning a polymer material over substantially all of a crown. The method can comprise positioning a spine distal end of a spine in a portion of the polymer material and proximal the crown so as to define a gap between the spine and the crown. The method can comprise heating the polymer material. The method can comprise, in response to the heating, shrinking the polymer material to cover the spine distal end and substantially all the crown.

### BRIEF DESCRIPTION OF THE DRAWINGS

FIG. 1 is a schematic pictorial illustration of a medical system including a medical probe whose distal end has a basket assembly with electrodes coupled together, in accordance with an embodiment of the disclosed technology;
FIG. 2 is a perspective view of an expandable basket assembly in a deployed position, in accordance with the disclosed technology;
FIG. 3A is a detail perspective of a spine in a delivery position, in accordance with the disclosed technology;
FIG. 3B is a detail perspective of the spine in a the deployed position, in accordance with the disclosed technology;
FIG. 4A is a detail side view of the spine in the delivery position, in accordance with the disclosed technology;
FIG. 4B is a detail side view of the spine in the deployed position, in accordance with the disclosed technology;
FIG. 5 is a detail side view of an exemplary hinge interconnecting a spine and a crown portion, in accordance with the disclosed technology;
FIG. 6 is a perspective view of a portion of the expandable basket assembly with the exemplary hinge and an exemplary coupling member, in accordance with the disclosed technology;
FIG. 7 is a perspective view of a portion of the expandable basket assembly with the exemplary hinge and another exemplary coupling member, in accordance with the disclosed technology;
FIG. 8 is a perspective view of a portion of the expandable basket assembly with another exemplary hinge, in accordance with the disclosed technology;
FIG. 9 is a detail side view of the exemplary hinge of FIG. 8 interconnecting the spine and the crown portion, in accordance with the disclosed technology;
FIG. 10 is a detail side view of another exemplary hinge, in accordance with the disclosed technology;
FIG. 11 is a detail side of another exemplary hinge, in accordance with the disclosed technology;
FIG. 12A is a side schematic view of an expandable basket assembly including a plug and force sensor in a first position, in accordance with the disclosed technology;
FIG. 12B is a side schematic view of an expandable basket assembly including a plug and force sensor in a second position, in accordance with the disclosed technology;
FIG. 12C is a side schematic view of an expandable basket assembly including a plug and force sensor in a third position, in accordance with the disclosed technology;
FIG. 13 is a flow chart of a method of constructing a j oint of an expandable basket assembly, in accordance with the disclosed technology; and
FIG. 14 is a flow chart of another method of constructing a joint of an expandable basket assembly, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" or "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, although the tubular structures may be generally illustrated as a substantially right cylindrical structure, the tubular structures may have a tapered or curved outer surface without departing from the scope of the present disclosure.

Example systems, methods, and devices of the present disclosure may be particularly suited for IRE ablation of cardiac tissue to treat cardiac arrhythmias. Ablative energies are typically provided to cardiac tissue by electrodes which can deliver ablative energy alongside the tissue to be ablated. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

Manufacturing and constructing of a medical probe capable of IRE as discussed above will now be discussed. The solution of this disclosure includes systems and methods for constructing a basket assembly. By coupling a plurality of spine struts with hinges to a crown of the distal tip, the basket assembly can be configured to transition between a deployed position and a delivery position. Furthermore, the hinges enable the basket assembly to form a more compact shape when in a delivery position. Further still, the hinges reduce the stress concentrations which can be present in spine, thereby reducing the likelihood that the spines may break when flexed.

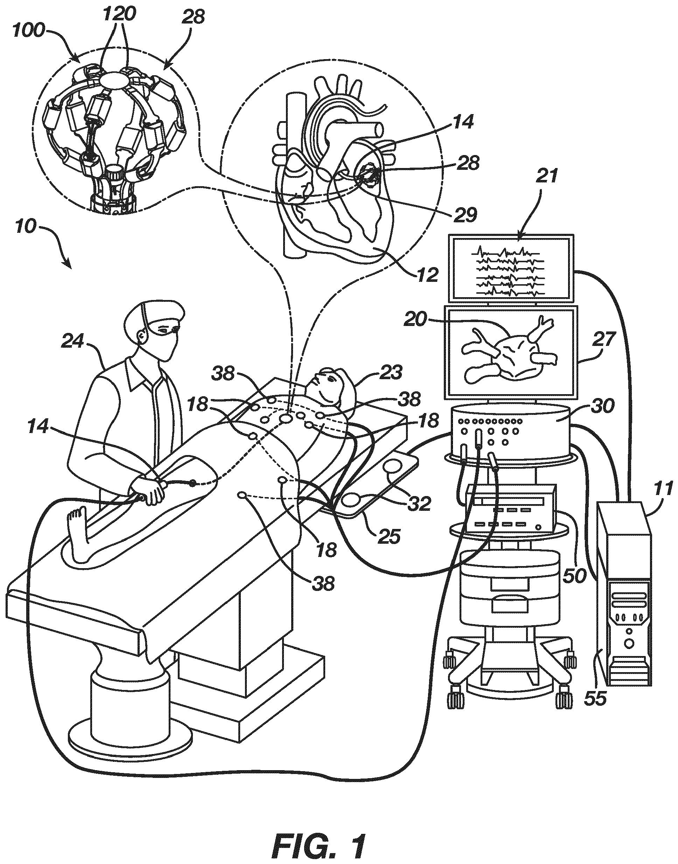

Reference is made to FIG. 1 showing an example catheter-based electrophysiology mapping and ablation system 10. System 10 includes multiple medical probes (e.g., catheters), which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of medical probes can be inserted into the delivery sheath catheter so as to arrive at the desired location. The plurality of medical probes may include catheters dedicated for sensing Intracardiac Electrogram (IEGM) signals, catheters dedicated for ablating and/or catheters dedicated for both sensing and ablating. An example medical probe 14 that is configured for sensing IEGM is illustrated herein. Physician 24 brings a distal tip 28 of medical probe 14 into contact with the heart wall for sensing a target site in heart 12. For ablation, physician 24 would similarly bring a distal end of an ablation catheter to a target site for ablating.

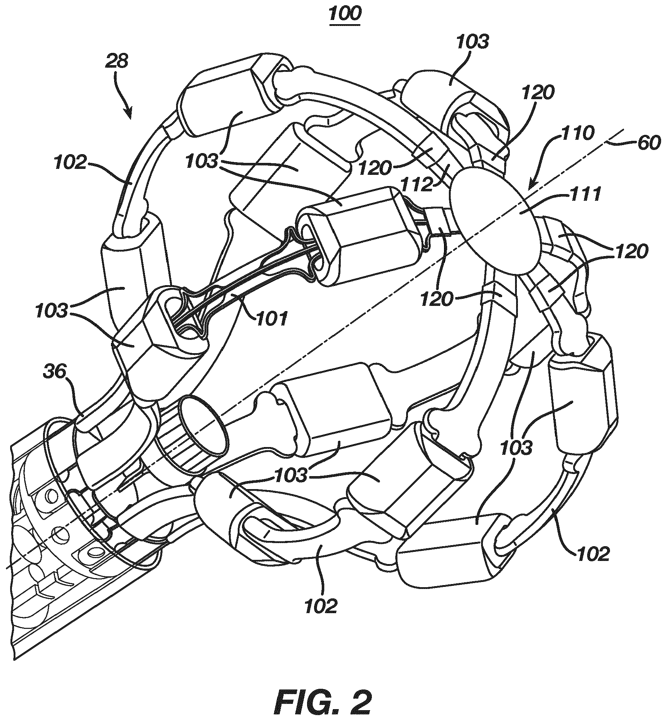

Medical probe 14 can be an exemplary catheter that includes one and preferably multiple electrodes 103 (FIG. 2) optionally distributed over a plurality of spines 101 (with insulative jackets 102 provided therearound) at distal tip 28 and configured to sense the IEGM signals. The medical probe 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of medical probe 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

FIG. 2 is a perspective view of the distal tip 28, in accordance with the disclosed technology. The distal tip 28 may be configured as an expandable basket assembly 100 (also referred to herein as an "end effector" and a "basket assembly"). The basket assembly 100 can be connected to a tubular shaft 36 that defines a longitudinal axis 60. As shown in FIG. 2, the basket assembly 100 can include a plurality of spines 101 that extend along (i.e., are substantially parallel to) the longitudinal axis 60 in a delivery position (i.e., a collapsed position) and that bow radially outward from the longitudinal axis 60 when in a deployed position (i.e., an expanded position in which they form an approximately spherical or approximately oblate-spheroid shape).

The spines 101 can have insulative jackets 102 provided thereover on certain portions. The spines 101 can include a first material. In some examples, the first material can include nitinol, cobalt chromium, stainless steel, titanium, or another resilient biocompatible material such as a polymer material.

The spines 101 can further include a plurality of electrodes 103 disposed thereon that can be configured for mapping of electrophysiological signals and/or ablation of tissue. The electrodes 103 can be any type of electrode configured including, but not limited to, electrodes suitable for sensing electrophysiological signals, ablation, position sensing, reference electrodes, etc. The electrodes 103 can be ring-type electrodes, bulging-type electrodes, rectangular electrodes, circular electrodes, etc. Furthermore, the electrodes 103 can be configured to deliver electrical pulses for irreversible electroporation, the pulses having a peak voltage of at least 900 volts (V).

The spines 101 can be attached, and one end thereof, to the distal end of a tubular shaft 36 and, at an opposing end thereof, to a crown 110 in a manner that permits them to move between the delivery position and the deployed position relative to the longitudinal axis 60. As used herein, a "crown" refers to a structure that functions as an attachment point for one or more spines 101 at a distal end thereof. In some examples, the crown 110 can also include the first material (e.g., nitinol). The crown 110 can be configured in a number of ways, in accordance with the disclosed technology. In some examples, the crown 110 can include a central crown hub 111 and a plurality of projections 112 protruding away from the hub. The projections 112 can be approximately coplanar with the central hub 111. In other examples, the crown 110 can exclude the projections 112.

The joint between each spine 101 and the crown 110 is a region that flexes in response to movement of the basket assembly 100 between the delivery position and the deployed position. In order to reduce or eliminate the fatigue that occurs at this joint, the basket assembly 100 can further include a hinge 120 provided between each spine 101 and the crown 110 to couple them together.

Each hinge 120 can include a second material that is different from the first material of the spine 101. In some examples, the second material comprises a flexible material capable of withstanding repeated bending without experiencing fatigue and/or failure. For example, the second material can include, but is not limited to, a polymer, e.g., polyolefin, a polymer metal, or polyester. Other exemplary second materials are provided below.

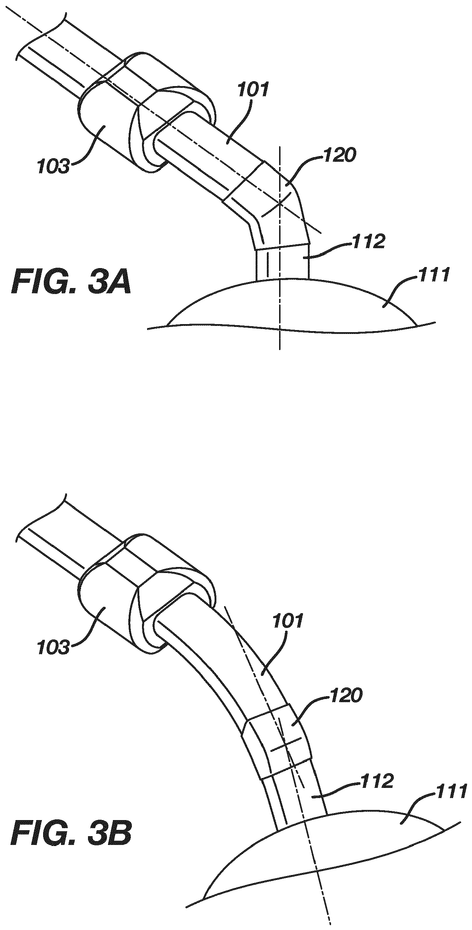

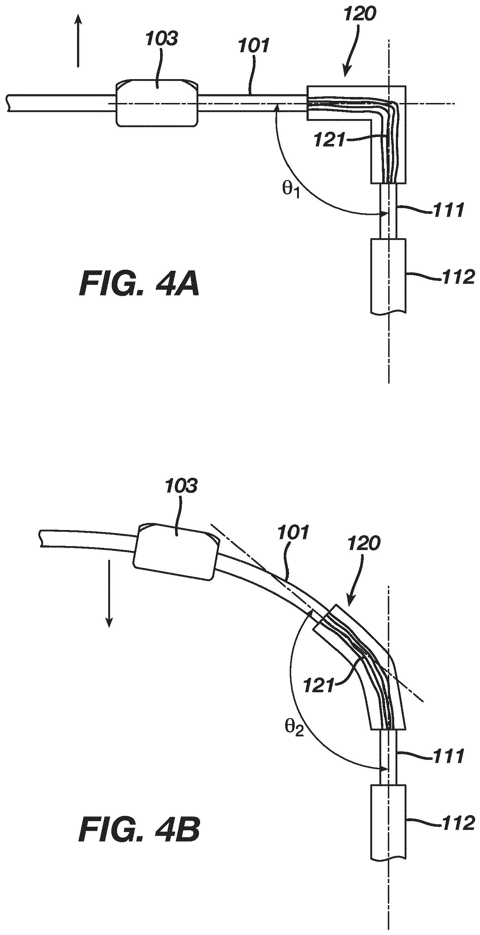

FIGs. 3A and 3B are detail perspective views depicting a spine 101 flexing between a delivery position (FIG. 3A) and a deployed position (FIG. 3B). FIGs. 4A and 4B are detail side views depicting the same. As shown, a spine distal end couples with one side the hinge 120 and a crown spine portion couples with the other side of the hinge 120. In some examples, each projection 112 can include a width at a proximal end thereof which is approximately equal to a width of the spine distal end.

In some examples, each hinge 120 can couple to a respective projection 112 of the crown. In other examples, the hinge 120 can couple to the crown hub 111 (e.g., when some or all of the projections 112 are omitted). As denoted by the directional arrows in FIGs. 4A and 4B, when the spines 101 are bowed outwardly (to the position shown in FIG. 4B) and the spine rotates from a first angle θ₁ to a second, greater angle θ₂ relative to the crown 110. In some examples, the first angle θ₁ can be approximately 90 degrees. In some examples, θ₂ can range anywhere between approximately 90 and approximately 180 degrees. In some examples, θ₂ can range between approximately 90 and approximately 130 degrees.

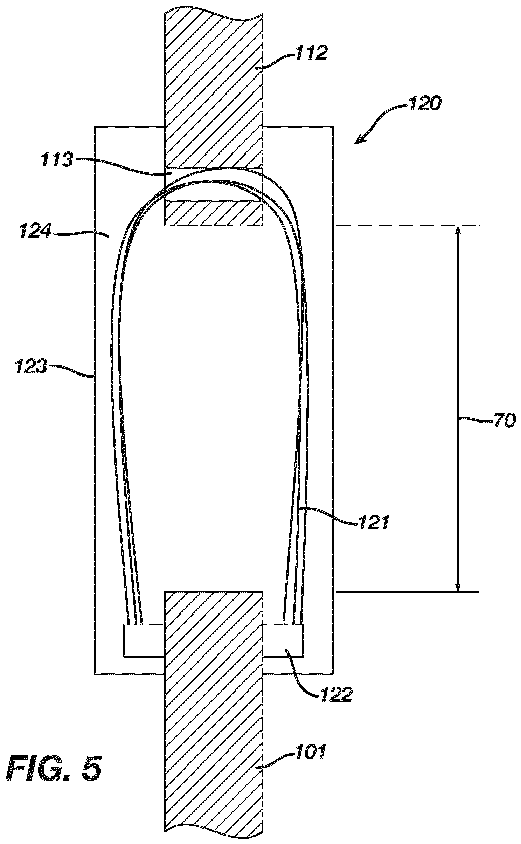

FIG. 5 is a detail cutaway view depicting an exemplary hinge 120, in accordance with the disclosed technology. As shown, the spine distal end and the crown spine portion (e.g., the crown protrusion 112) are separated by a hinge gap 70. The gap 70 correlates to the maximum offset the crown 111 can have when compressed. In some examples, the gap 70 can be a distance of approximately 1-5 millimeters. In some examples, the gap 70 can be approximately equal to a thickness of the spine 101 which would allow the spines 101 to fold without interfering with each other. This hinge gap 70 is covered by at least the hinge 120. In some examples, the hinge 120 is formed in such a way that it allows the crown spine portion and spine distal end to move towards and away form one another (e.g., it can be formed from a flexible material that can be compressed and/or expanded when a force is applied to it).

In some examples, as exemplified in FIGs. 4A-5, the hinge 120 can include a cap 123. In some examples, the cap 123 can comprise a shrinkable tube (e.g., heat shrink). The shrinkable tube can be disposed over portions of the crown 110 and the spine 101 to cover any sharp edges that may otherwise be exposed. In some examples, the cap 123 can be formed as a nitinol tube, a stainless spring steel tube, PEEK tube, or laminated material over the hinge gap 70.

Making further reference to FIG. 5, a coupling member 121 can extend at least partially through the hinge 120 to prevent the spine 101 and crown 110 from pulling away from each other greater than the gap 70. The coupling member 121 can be coupled to the spine 101 and crown 111 in various ways. For example, one or more anchor points 122 can be provided on the distal end of the spine 101 to which ends of the coupling member 121 are mounted to (e.g., via an adhesive or a mechanical connection). Further, an opening 113 can be defined in the crown 110 (e.g., defined in the projection 112) through which the coupling member 112 is routed. Alternatively, this coupling arrangement can be reversed.

Further, an adhesive 124 can be filled in the hinge 120 to retain it at least partially to the spine 101 and crown 110. In some examples, the hinge 120 can include the coupling member 121 and/or the adhesive 124.

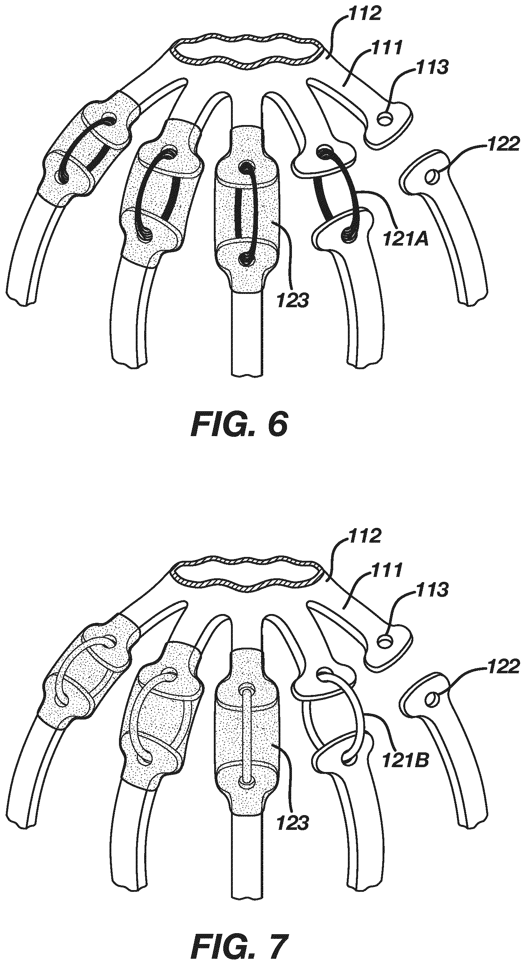

In some examples, the coupling member 121, such as depicted in FIG. 6, can include a flexible material 121A, such as, but not limited to yarn (e.g., polyester yarn), Nitinol, a manufactured fiber spun from a liquid-crystal polymer, PEBAX^{©}, molded thermal plastic couplings such as a nylon base, stainless steel wire, and carbon-based fibers. In some examples, such as depicted in FIG. 7, the coupling member 121 can include a plurality of links 121B that are flexibly interconnected with one another.

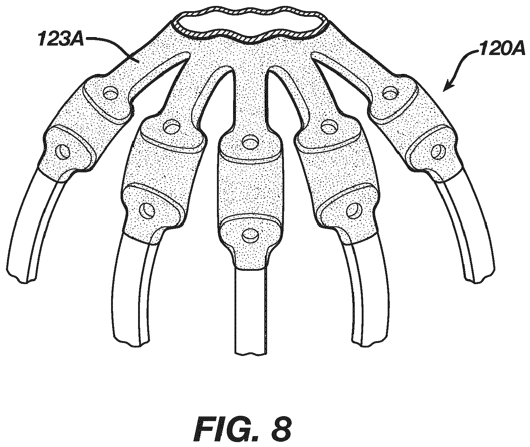

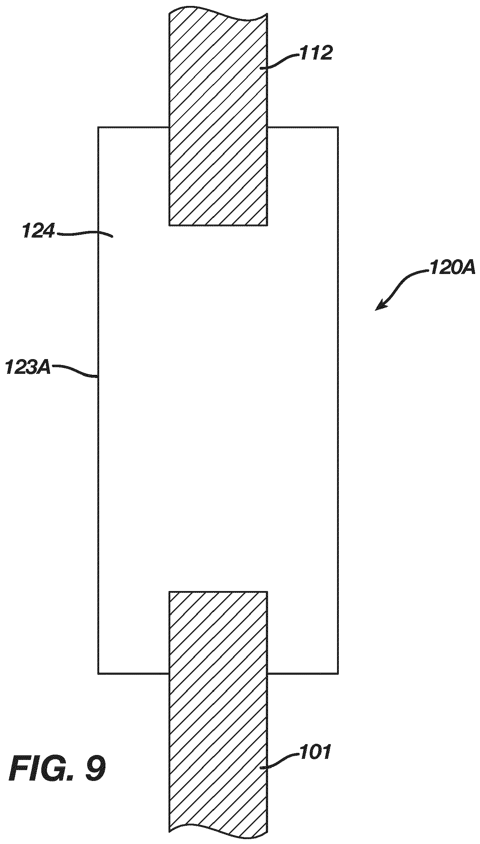

Turning to FIGs. 8 and 9, rather than providing a cap 123 at each joint between spine 101 and crown 110, a hinge 120A can be provided that substantially covers the crown and all of the spine ends. The hinge 120A can include a cap 123A that is overmolded on the crown 110 and end portions of the spines 101. Similar to the cap 123 previously described, this cap 123A can also have adhesive 124 filled therewithin.

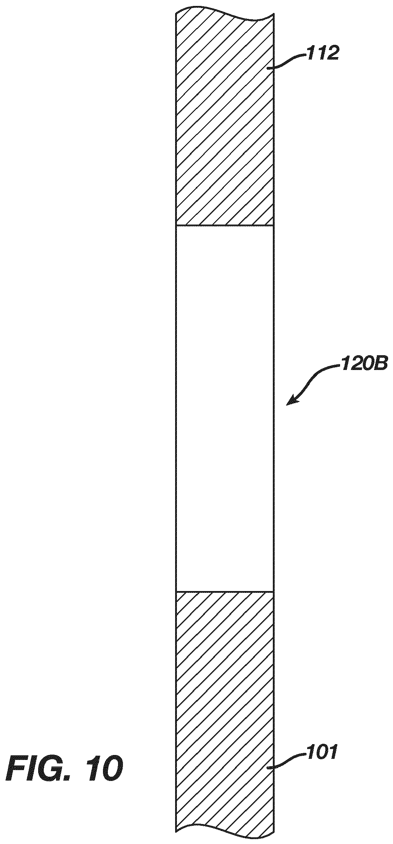

FIG. 10 is a detail side view of another exemplary hinge 120B. In this example, rather than covering ends of the crown 110 and spine 101, the hinge 120B can be formed from a flexible material and directly connected to distal tips of the crown 110 (e.g., a distal tip of the projection 112) and the spine 101.

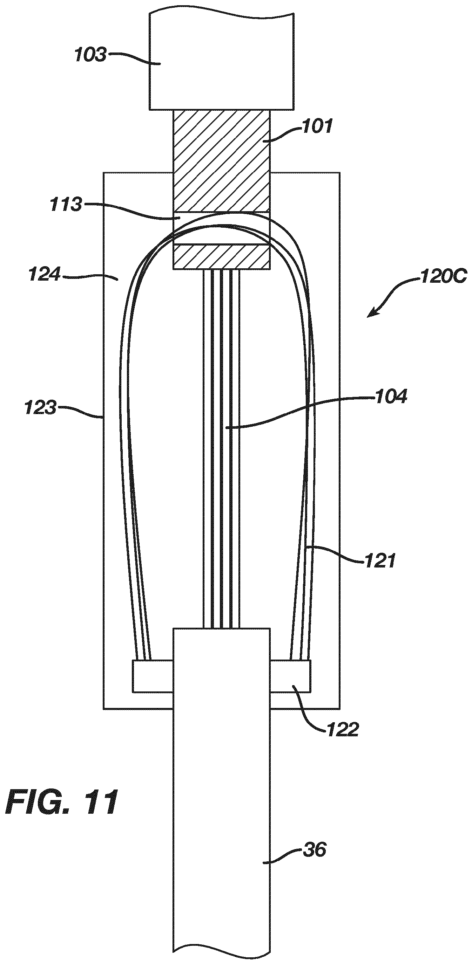

Making reference to FIG. 11, another exemplary hinge 120C is shown. It can be structured in a similar manner as that of the other examples described (e.g., the one described with respect to FIG. 5). However, rather than coupling the crown 110 and spine 101 together, the hinge 120C can also be used at other points on the basket assembly 100, such as to connect the spine 101 and the tubular shaft 36. In this example, various electrical connections 104 (e.g., wiring) can be routed through the hinge 120C to electrically connect the electrodes 103 with the electrophysiological equipment.

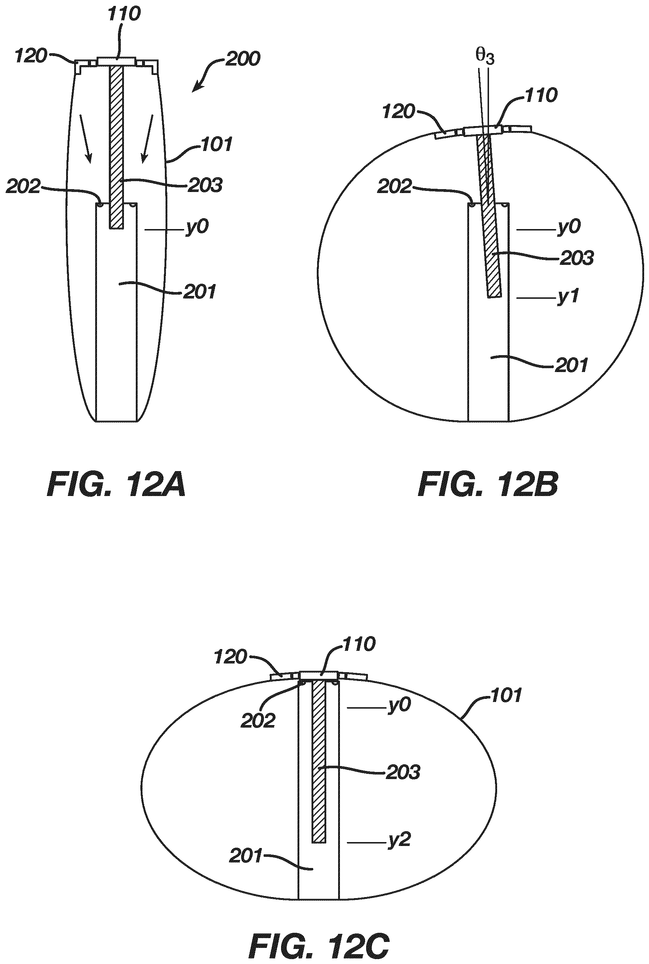

In addition to the above-described exemplary hinges 120, the present disclosure provides ways in which to measure a force applied by the crown 110 to tissue in the patient 23. FIGs. 12A-12C depict side schematic views of an expandable basket assembly 200 including a plug 201 and force sensor 203 disposed at various positions. The plug 201 can be coupled to the tubular shaft 36 at a proximal end and may include irrigation holes 202 at a distal end thereof. At the distal end, a force sensor 203 can extend out in a slidable and rotatable manner and couple to a bottom side of the crown 110. As shown from FIG. 12A to 12B and 12C, when in the delivery position, the force sensor 203 can extend a distance y0 into the plug 201 and be substantially co-axial with the longitudinal axis 60.

When the spines 101 are expanded, the force sensor 203 is urged down into the plug 201 a distance of y1. Further, when the crown 110 or spines 101 contact tissue, that can cause the crown to angularly deflect, as denoted by angle θ₃ in FIG. 12B. Further pressing on, for example, the crown 110 can further push the force sensor 203 within the plug 201 at a distance y2. When the crown 110 is pressed all the way down onto the distal end of the plug 201, the irrigation holes 202 can be blocked, as shown in FIG. 12C. The measured distances y0, y1, and y2 and angles θ₃ can be used to calculate various parameters associated with the basket assembly 200, such as the force and vector of which it is contacting a patient's tissue.

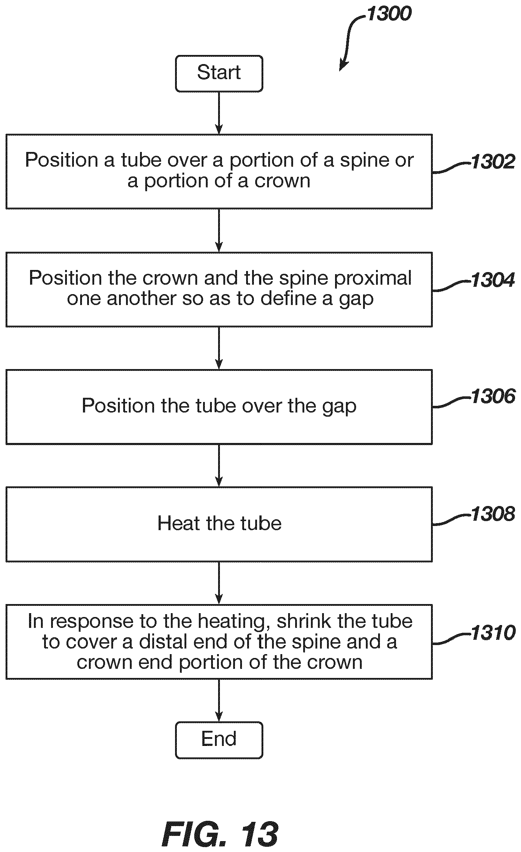

FIG. 13 is a flow chart of a method 1300 of constructing a joint of an expandable basket assembly, in accordance with the disclosed technology. The basket assembly can include all of the same features as the basket assemblies described further herein. The method 1300 can include positioning 1302 a tube over a portion of a spine or a portion of a crown. The method 1300 can include positioning 1304 the crown and the spine proximal one another so as to define a gap. The method 1300 can include positioning 1306 the tube over the gap. The method 1300 can include heating 1308 the tube. The method 1300 can include, in response to the heating, shrinking 1310 the tube to cover a spine distal end of the spine and a crown end portion of the crown. In some examples, the method can include filling the tube with adhesive. In some examples, the method 1300 can include coupling a coupling member to the spine and to the crown. In some examples of the method 1300, the coupling member can include polyester yarn or a plurality of links flexibly that are interconnected. In some examples, the method 1300 can further include defining a hole in the crown end portion.

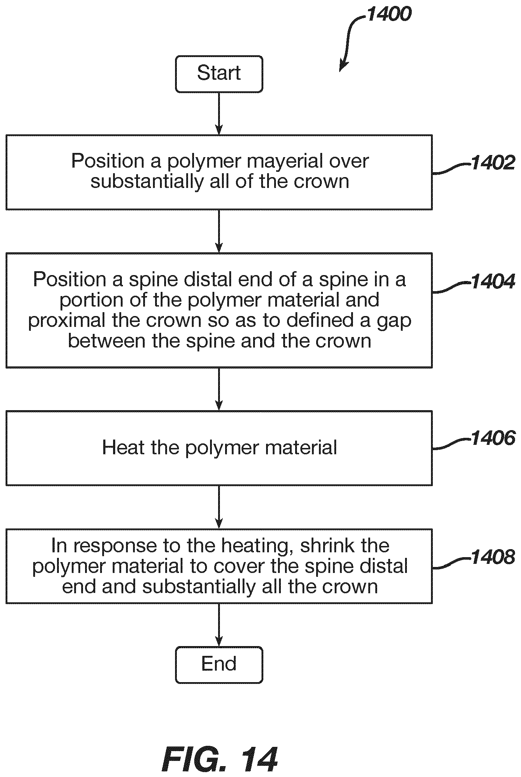

FIG. 14 is a flow chart of another method 1400 of constructing a joint of an expandable basket assembly, in accordance with the disclosed technology. The basket assembly can include all of the same features as the basket assemblies described further herein. The method 1400 can include positioning 1402 a polymer material over substantially all of a crown. The method 1400 can include positioning 1404 a spine distal end of a spine in a portion of the polymer material and proximal the crown so as to define a predetermined gap between the spine and the crown. The method 1400 can include heating 1406 the polymer material. The method 1400 can include, in response to the heating, shrinking 1408 the polymer material to cover the spine distal end and substantially all the crown. In some examples, the method 1400 can include filling the polymer material with adhesive.

As will be appreciated by one skilled in the art, methods 1300 and 1400 can include any of the various features of the disclosed technology described herein and can be varied depending on the particular configuration. Thus, methods 1300 and 1400 should not be construed as limited to the particular steps and order of steps explicitly described herein.

The disclosed technology described herein can be further understood according to the following clauses:
Clause 1: A joint of an end effector of a medical probe, the end effector comprising: a crown; at least one spine extending along a longitudinal axis, comprising a deployed position and delivery position relative to the longitudinal axis, and comprising a first material; and at least one hinge coupling the at least one spine to the crown, the at least one hinge comprising a second material different from the first material.
Clause 2: The joint of an end effector of clause 1, the at least one spine being expandable from the delivery position, where the at least one spine is substantially parallel to the longitudinal axis, to the deployed position, where the at least one spine bows outwardly from the longitudinal axis, the at least one hinge flexing in response to expansion between the delivery and the deployed positions of the at least one spine.
Clause 3: The joint of an end effector of any one of clauses 1-2, the at least one spine comprising a spine distal end, and the crown comprises at least one crown spine portion, the at least one hinge coupling the spine distal end to the at least one crown spine portion.
Clause 4: The joint of an end effector of clause 3, the crown spine portion and the spine distal end being separated by a hinge gap, the hinge gap covered at least by the hinge.
Clause 5: The joint of an end effector of clause 4, the hinge gap comprising a distance in the range of approximately one to five millimeters.
Clause 6: The joint of an end effector of clause 4 or 5, the second material of the at least one hinge permitting the at least one crown spine portion and the spine distal end to move towards and away from one another.
Clause 7: The joint of an end effector of any one of clauses 1-6, the crown comprising a central hub, and the crown spine portion defining at least one projection protruding away from the hub.
Clause 8: The joint of an end effector of clause 7, the at least one projection comprising a width at a proximal end of the at least one projection which is approximately equal to a width of the spine distal end.
Clause 9: The joint of an end effector of clause 7 or 8, the at least one projection being approximately coplanar with the central hub.
Clause 10: The joint of an end effector of any one of clauses 1-9, further comprising at least one coupling member extending at least partially through the at least one hinge.
Clause 11: The joint of an end effector of clause 10, the crown defining at least one opening, the at least one coupling member extending through the at least one opening to couple the at least one coupling member to the crown.
Clause 12: The joint of an end effector of clause 10 or 11, the at least one spine comprising at least one anchor point to which an end of the at least one coupling member is coupled to.
Clause 13: The joint of an end effector of any one of clauses 10-12, the at least one spine comprising two anchor points to which ends of the at least one coupling member are coupled to.
Clause 14: The joint of an end effector of any one of clauses 10-13, the at least one coupling member comprising polyester yarn.
Clause 15: The joint of an end effector of any one of clauses 11-14, the at least one coupling member comprising a plurality of links flexibly interconnected.
Clause 16: The joint of an end effector of any one of clauses 1-9, the second material comprising polyester yarn.
Clause 17: The joint of an end effector of any one of clauses 1-9, the hinge comprising a plurality of links flexibly interconnected.
Clause 18: The joint of an end effector of any one of clauses 1-17, the at least one hinge comprising a shrinkable tube.
Clause 19: The joint of an end effector of clause 18, end portions of the shrinkable tube being disposed over a portion of the crown and a portion of the at least one spine.
Clause 20: The joint of an end effector of clause 18 or 19, the second material comprising polyolefin.
Clause 21: The joint of an end effector of any one of clauses 1-17, at least a portion of the at least one hinge being overmolded on the crown and an end portion of the at least one spine.
Clause 22: The joint of an end effector of clause 21, the second material comprising a shrinkable material.
Clause 23: The joint of an end effector of any one of clauses 1-22, further comprising an adhesive retaining the at least one hinge in place.
Clause 24: The joint of an end effector of any one of clauses 1-23, the first material comprising nitinol.
Clause 25: The joint of an end effector of any one of clauses 1-24, the crown comprising nitinol.
Clause 26: The joint of an end effector of any one of clauses 1-25, the at least one spine having at least one electrode coupled thereto.
Clause 27: The joint of an end effector of clause 26, the at least one electrode being configured to electrophysiologically map a tissue, ablate the tissue, or both map and ablate the tissue.
Clause 28: A medical probe comprising: a tubular shaft defining a longitudinal axis and having a tubular shaft end; and an end effector coupled to the tubular shaft end, the end effector comprising: a crown; at least one spine extending along a longitudinal axis, comprising a deployed position and delivery position relative to the longitudinal axis, and comprising a first material; and at least one hinge coupling the at least one spine to the crown, the at least one hinge comprising a second material different from the first material.
Clause 29: The medical probe of clause 28, the at least one spine being expandable from the delivery position, where the at least one spine is substantially parallel to the longitudinal axis, to the deployed position, where the at least one spine bows outwardly from the longitudinal axis, the at least one hinge flexing in response to expansion between the delivery and the deployed positions of the at least one spine.
Clause 30: The medical probe of clause 28 or 29, the at least one spine comprising a spine distal end, and the crown comprises at least one crown spine portion, the at least one hinge coupling the spine distal end to the at least one crown spine portion.
Clause 31: The medical probe of clause 30, the crown spine portion and the spine distal end being separated by a hinge gap, the hinge gap covered at least by the hinge.
Clause 32: The medical probe of clause 31, the hinge gap comprising a distance in the range of approximately one to five millimeters.
Clause 33: The medical probe of clause 31 or 32 the second material of the at least one hinge permitting the at least one crown spine portion and the spine distal end to move towards and away from one another.
Clause 34: The medical probe of any one of clauses 28-33, the crown comprising a central hub, and the crown spine portion defining at least one projection protruding away from the hub.
Clause 35: The medical probe of clause 34, the at least one projection comprising a width at a proximal end of the at least one projection which is approximately equal to a width of the spine distal end.
Clause 36: The medical probe of clause 34 or 35, the at least one projection being approximately coplanar with the central hub.
Clause 37: The medical probe of any one of clauses 28-36, further comprising at least one coupling member extending at least partially through the at least one hinge.
Clause 38: The medical probe of clause 37, the crown defining at least one opening, the at least one coupling member extending through the at least one opening to couple the at least one coupling member to the crown.
Clause 39: The medical probe of clause 37 or 38, the at least one spine comprising at least one anchor point to which an end of the at least one coupling member is coupled to.
Clause 40: The medical probe of any one of clauses 37-39, the at least one spine comprising two anchor points to which ends of the at least one coupling member are coupled to.
Clause 41: The medical probe of any one of clauses 37-40, the at least one coupling member comprising polyester yarn.
Clause 42: The medical probe of any one of clauses 38-41, the at least one coupling member comprising a plurality of links flexibly interconnected.
Clause 43: The medical probe of any one of clauses 28-36, the second material comprising polyester yarn.
Clause 44: The medical probe of any one of clauses 28-36, the hinge comprising a plurality of links flexibly interconnected.
Clause 45: The medical probe of any one of clauses 28-44, the at least one hinge comprising a shrinkable tube.
Clause 46: The medical probe of clause 45, end portions of the shrinkable tube being disposed over a portion of the crown and a portion of the at least one spine.
Clause 47: The medical probe of clause 45 or 46, the second material comprising polyolefin.
Clause 48: The medical probe of any one of clauses 28-44, at least a portion of the at least one hinge being overmolded on the crown and an end portion of the at least one spine.
Clause 49: The medical probe of clause 48, the second material comprising a shrinkable material.
Clause 50: The medical probe of any one of clauses 28-49, further comprising an adhesive retaining the at least one hinge in place.
Clause 51: The medical probe of any one of clauses 28-50, the first material comprising nitinol.
Clause 52: The medical probe of any one of clauses 28-51, the crown comprising nitinol.
Clause 53: The medical probe of any one of clauses 28-52, the at least one spine having at least one electrode coupled thereto.
Clause 54: The medical probe of clause 53, the at least one electrode being configured to electrophysiologically map a tissue, ablate the tissue, or both map and ablate the tissue.
Clause 55: The medical probe of any one of clauses 28-54, further comprising a central plug disposed proximal the at least one spine.
Clause 56: The medical probe of clause 55, the central plug defining at least one hole, the at least one spine or the crown being configured to block the at least one hole when the at least one spine is in the deployed position.
Clause 57: The medical probe of clause 56, the at least one spine or the crown being configured to engage a distal tip of the central plug to block the at least one hole.
Clause 58: The medical probe of clause 57, the at least one spine or the crown being configured to engage a distal tip of the central plug when the at least one spine is in the deployed position.
Clause 59: The medical probe of any one of clauses 55-59, further comprising a force sensor coupled to the central plug, the force sensor being configured to measure a force applied by the at least one spine or the crown to the distal tip.
Clause 60: A medical probe comprising: a tubular shaft defining a longitudinal axis and having a tubular shaft end; and an end effector comprising: at least one spine extending along a longitudinal axis, comprising a deployed position and delivery position relative to the longitudinal axis, and comprising a first material; and at least one hinge coupling the at least one spine to the tubular shaft end, the at least one hinge comprising a second material different from the first material.
Clause 61: The medical probe of clause 60, the at least one spine having at least one electrode coupled thereto.
Clause 62: The medical probe of clause 60 or 61, comprising wiring extending through the at least one hinge.
Clause 63: A method of constructing a joint of an end effector, the method comprising: positioning a tube over a portion of a spine or a portion of a crown; positioning the crown and the spine proximal one another so as to define a gap; positioning the tube over the gap; heating the tube; and in response to the heating, shrinking the tube to cover a spine distal end of the spine and a crown end portion of the crown.
Clause 64: The method of clause 63, further comprising: filling the tube with adhesive.
Clause 65: The method of clause 63 or 64, further comprising: coupling a coupling member to the spine and to the crown.
Clause 66: The method of clause 65, wherein the coupling member comprises polyester yarn or a plurality of links flexibly that are interconnected.
Clause 67: The method of any one of clauses 63-66, further comprising: defining a hole in the crown end portion.
Clause 68: A method of constructing a joint of an end effector, the method comprising: positioning a polymer material over substantially all of a crown; positioning a spine distal end of a spine in a portion of the polymer material and proximal the crown so as to define a gap between the spine and the crown; heating the polymer material; and in response to the heating, shrinking the polymer material to cover the spine distal end and substantially all the crown.
Clause 69: The method of clause 68, further comprising: filling the polymer material with adhesive.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A joint of an end effector of a medical probe, the end effector comprising:
a crown;
at least one spine extending along a longitudinal axis, comprising a deployed position and delivery position relative to the longitudinal axis, and comprising a first material; and
at least one hinge coupling the at least one spine to the crown, the at least one hinge comprising a second material different from the first material.

2. The joint of an end effector of claim 1, the at least one spine being expandable from the delivery position, where the at least one spine is substantially parallel to the longitudinal axis, to the deployed position, where the at least one spine bows outwardly from the longitudinal axis, the at least one hinge flexing in response to expansion between the delivery and the deployed positions of the at least one spine.

3. The joint of an end effector of claim 1 or claim 2, the at least one spine comprising a spine distal end, and the crown comprises at least one crown spine portion, the at least one hinge coupling the spine distal end to the at least one crown spine portion, the crown spine portion and the spine distal end being separated by a hinge gap, the hinge gap covered at least by the hinge.

4. The joint of an end effector of any preceding claim, the crown comprising a central hub, and the at least one crown spine portion defining at least one projection protruding away from the hub, the at least one projection (i) comprising a width at a proximal end of the at least one projection which is approximately equal to a width of the spine distal end, or (ii) being approximately coplanar with the central hub.

5. The joint of an end effector of any preceding claim, further comprising at least one coupling member extending at least partially through the at least one hinge, (i) the crown defining at least one opening, the at least one coupling member extending through the at least one opening to couple the at least one coupling member to the crown, and the at least one spine comprising at least one anchor point to which an end of the at least one coupling member is coupled to, or (ii) the at least one coupling member comprising polyester yarn and/or a plurality of links flexibly interconnected.

6. The joint of an end effector of any preceding claim, the at least one hinge comprising a shrinkable tube.

7. The joint of an end effector of any preceding claim, the first material and/or the crown comprising nitinol.

8. The joint of an end effector of any preceding claim, the at least one spine having at least one electrode coupled thereto such that the at least one electrode is configured to electrophysiologically map a tissue, ablate the tissue, or both map and ablate the tissue.

9. A medical probe comprising:
a tubular shaft defining a longitudinal axis and having a tubular shaft end; and
an end effector coupled to the tubular shaft end, the end effector comprising:
a crown;
at least one spine extending along the longitudinal axis, comprising a deployed position and delivery position relative to the longitudinal axis, and comprising a first material; and
at least one hinge coupling the at least one spine to the crown, the at least one hinge comprising a second material different from the first material.

10. The medical probe of claim 9, the at least one spine being expandable from the delivery position, where the at least one spine is substantially parallel to the longitudinal axis, to the deployed position, where the at least one spine bows outwardly from the longitudinal axis, the at least one hinge flexing in response to expansion between the delivery and the deployed positions of the at least one spine.

11. The medical probe of claim 9 or claim 10, the at least one spine comprising a spine distal end, and the crown comprises at least one crown spine portion, the at least one hinge coupling the spine distal end to the at least one crown spine portion, the crown spine portion and the spine distal end being separated by a hinge gap, the hinge gap covered at least by the hinge.

12. The medical probe of any of claims 9 to 11, the crown comprising a central hub, and the at least one crown spine portion defining at least one projection protruding away from the hub, the at least one projection being approximately coplanar with the central hub.

13. The medical probe of claim 9, further comprising at least one coupling member extending at least partially through the at least one hinge, the crown defining at least one opening, the at least one coupling member being flexible and extending through the at least one opening to couple the at least one coupling member to the crown, the at least one spine comprising at least one anchor point to which an end of the at least one coupling member is coupled to.

14. The medical probe of any of claims 9 to 13, the at least one spine having at least one electrode coupled thereto such that the at least one electrode is configured to electrophysiologically map a tissue, ablate the tissue, or both map and ablate the tissue.

15. The medical probe of any of claims 9 to 14, further comprising a central plug disposed proximal the at least one spine.

16. A medical probe comprising:
a tubular shaft defining a longitudinal axis and having a tubular shaft end; and
an end effector comprising:
at least one spine extending along the longitudinal axis, comprising a deployed position and delivery position relative to the longitudinal axis, and comprising a first material; and
at least one hinge coupling the at least one spine to the tubular shaft end, the at least one hinge comprising a second material different from the first material.

17. The medical probe of claim 16, the at least one spine having at least one electrode coupled thereto, and/or comprising wiring extending through the at least one hinge.
